# EUROPEAN PATENT SPECIFICATION

(11) **EP 0 937 097 B1**
(45) Date of publication and mention of the grant of the patent: **16.08.2001**
(21) Application number: 97947374.1
(22) Date of filing: 06.11.1997
(51) Int. Cl.: C07H 21/00

(54) **COMPOSITIONS AND METHODS FOR IMMOBILIZING NUCLEIC ACIDS TO SOLID SUPPORTS**
ZUSAMMENSETZUNGEN UND VERFAHREN ZUR IMMOBILISIERUNG VON NUKLEINSÄURE AUF FESTTRÄGERN
COMPOSITIONS ET PROCEDES D'IMMOBILISATION D'ACIDES NUCLEIQUES SUR DES SUPPORTS SOLIDES

(30) Priority: 06.11.1996 US 746036; 23.01.1997 US 786988; 23.01.1997 US 787639; 19.09.1997 US 933792
(43) Date of publication of application: 25.08.1999
(73) Proprietor: SEQUENOM, INC., San Diego, California 92121 (US)
(72) Inventor: LOUGH, David, M., Eyemouth, Berwickshire TD14 55A (GB); KÖSTER, Hubert, La Jolla, CA 92037 (US)
(74) Representative: Baldock, Sharon Claire
(86) International application number: US9720194
(87) International publication number: WO9820019

(56) References cited:
- EP-A- 0 396 116
- EP-A- 0 455 905
- WO-A-93/06925
- US-A- 4 797 355
- ARSHADY R: "BEADED POLYMER SUPPORTS AND GELS I. MANUFACTURING TECHNIQUES" JOURNAL OF CHROMATOGRAPHY, vol. 586, no. 2, 22 November 1991, pages 181-197, XP000247968
- ARSHADY E: "BEADED POLYMER SUPPORTS AND GELS II. PHYSICO-CHEMICAL CRITERIA AND FUNCTIONALIZATION" JOURNAL OF CHROMATOGRAPHY, vol. 586, no. 2, 22 November 1991, pages 199-219, XP000247969
- PON R T ET AL: "DERIVATIZATION OF CONTROLLED PORE GLASS BEADS FOR SOLID PHASE OLIGONUCLEOTIDE SYNTHESIS" BIOTECHNIQUES, vol. 6, no. 8, 1 January 1988, pages 768-770, 773 - 775, XP000562920
- LUND V ET AL: "ASSESMENT OF METHODS FOR COVALENT BINDING OF NUCLEIC ACIDS TO MAGNETIC BEADS, DYNABEADSTM, AND THE CHARACTERISTICS OF THE BOUND NUCLEIC ACIDS IN HYBRIDIZATION REACTIONS" NUCLEIC ACIDS RESEARCH, vol. 16, no. 22, 1 January 1988, XP000195548
- TOSHIO HAYASHI ET AL: "IMMOBILIZATION OF THIOL PROTEASES ONTO POROUS POLY(VINYL ALOCOHOL) BEADS" POLYMER JOURNAL, vol. 25, no. 5, 15 May 1993, pages 489-497, XP000398929
- DAMHA M J ET AL: "AN IMPROVED PROCEDURE FOR DERIVATIZATION OF CONTROLLED-PORE GLASS BEADS FOR SOLID-PHASE OLIGONUCLEOTIDE SYNTHESIS" NUCLEIC ACIDS RESEARCH, vol. 18, no. 13, 11 July 1990, pages 3813-3821, XP000562627

## Description

### BACKGROUND OF THE INVENTION

In the fields of molecular biology and biochemistry, as well as in the diagnosis of diseases, nucleic acid hybridization has become a powerful tool for the detection, isolation, and analysis of specific oligonucleotide sequences. Typically, such hybridization assays utilize an oligodeoxynucleotide probe that has been immobilized on a solid support; as for example in the reverse dot blot procedure (Saiki et al. (1989) Proc. Natl. Acad. Sci. U.S.A. 86:6230). More recently, arrays of immobilized DNA probes attached to a solid surface have been developed for sequencing by hybridization (SBH) (Drmanac et al. (1989) Genomics 4:114-128), (Strezoska et al. (1991) Proc. Natl. Acad. Sci. U.S.A. 88:10089-10093). SBH uses an ordered array of immobilized oligodeoxynucleotides on a solid support. A sample of unknown DNA is applied to the array, and the hybridization pattern is observed and analyzed to produce many short bits of sequence information simultaneously. An enhanced version of SBH, termed positional SBH (PSBH), has been developed which uses duplex probes containing single-stranded 3'- or 5'-overhangs. (Broude et al. (1994) Proc. Natl. Acad. Sci. U.S.A. 91:3072-3076). It is now possible to combine a PSBH capture approach with conventional Sanger sequencing to produce sequencing ladders detectable, for example by gel electrophoresis (Fu et al. (1995) Proc. Natl. Acad. Sci. U.S.A. 92:10162-10166).

For the arrays used in these schemes, there are a number of criteria that must be met for successful performance. For example, the immobilized DNA must be stable and not desorb during hybridization, washing, or analysis. In addition, the density of the immobilized oligodeoxynucleotide must be sufficient for the ensuing analyses. There must be minimal non-specific binding of DNA to the surface. In addition, the immobilization process should not interfere with the ability of immobilized probes to hybridize. For the majority of applications, it is best for only one point of the DNA to be immobilized, ideally a terminus.

In recent years a number of methods for the covalent immobilization of DNA to solid supports have been developed which attempt to meet all the criteria listed above. For example, appropriately modified DNA has been covalently attached to flat surfaces functionalized with amino acids (see, e.g., Running et al. (1990) Biotechniques 8:276-277; Newton et al. (1993) Nucl. Acids. Res. 21:1155-1162; and Nikiforov et al. (1995) Anal. Biochem. 227:201-209) carboxyl groups (see, e.g. Zhang et al. (1991) Nucl. Acids Res. 19: 3929-3933), epoxy groups (see, e.g., Lamture et al. (1994)Nucl. Acids Res. 22:2121-2125; Eggers et al. (1994) BioTechniques 17:516-524) or amino groups (see, e.g., Rasmussen et al. (1991) Anal. Biochem. 198:138-142). Although many of these methods were successful for their respective applications, when used to link nucleic acids to two-dimensional (flat) supports, the density of the immobilized oligodeoxynucleotide is often insufficient for the ensuing analyses (see, e.g., Lamture (1994) Nucl. Acids Res. 22:2121-2125; Eggers et al. (1994) BioTechniques 17:516-524).

Thus, there is a need for improved methods for immobilization that provide higher densities of linked molecules for ensuing analyses. Therefore, it is an object herein to provide methods for preparing solid supports containing high densities of immobilized molecules, particularly nucleic acid molecules.

### SUMMARY OF THE INVENTION

Compositions containing at least one bead conjugated to a solid support and further conjugated to at least one molecule, particularly a nucleic acid are provided. The bead is formed from any suitable matrix material known to those of skill in the art, including those that are swellable and nonswellable. The solid support is any support known to those of skill in the art for use as a support matrix in chemical syntheses and analyses.

Preferably the bead is made of a material selected from materials that serve as solid supports for synthesis and for assays including but not limited to: silica gel, glass, magnet, polystyrene/1% divinylbenzene resins, such as Wang resins, which are Fmoc-amino acid-4-(hydroxymethyl)phenoxymethylcopoly(styrene-1 % divinylbenzene (DVD)) resin, chlorotrityl (2-chlorotritylchloride copolystyrene-DVB resin) resin, Merrifield (chloromethylated copolystyrene-DVB) resin metal, plastic, cellulose, cross-linked dextrans, such as those sold under the tradename Sephadex® (Pharmacia) and agarose gel, such as gels sold under the tradename Sepharose® (Pharmacia), which is a hydrogen bonded polysaccharide-type agarose gel, and other such resins and solid phase supports known to those of skill in the art. In a preferred embodiment, the bead is of a size in the range of about 0.1 to 500 *µ*m, more preferably about 1 to 100 *µ*m, in diameter.

The solid support is in any desired form, including, but not limited to: a bead, capillary, plate, membrane, wafer, comb, pin, a wafer with pits, an array of pits or nanoliter wells and other geometries and forms known to those of skill in the art.

Methods for conjugation of beads to supports are provided. In a preferred embodiment, a covalent amide bond is formed between the bead and the insoluble support. In a particularly preferred embodiment, the covalent amide bond is formed by reacting a carboxyl-functionalized bead with an amino-functionalized solid support; or a carboxyl-functionalized support with an amino-functionalized bead.

In a further aspect, the invention features methods for isolating target nucleic acids from a sample or reaction mixture by a conjugation means described herein. In a particularly preferred method, the nucleic acids are directly analyzed by mass spectrometry.

Kits containing reagents for performing the conjugations and thereby immobilizing nucleic acids to an insoluble support via a bead linker are also provided.

As compared to "flat" surfaces, beads linked to a solid support provide an increased surface area for immobilization of nucleic acids. Furthermore, by selecting a bead with the desired functionality, a practitioner can select a functionalization chemistry for immobilizing nucleic acids, which is different from the chemistry of the solid support.

The above and further features and advantages of the compositions and methods provided herein will be elucidated in the following Figures, Detailed Description and Claims.

### BRIEF DESCRIPTION OF THE DRAWINGS

Figure 1 is a schematic showing the covalent attachment of a bead to a solid support and DNA to the bead.

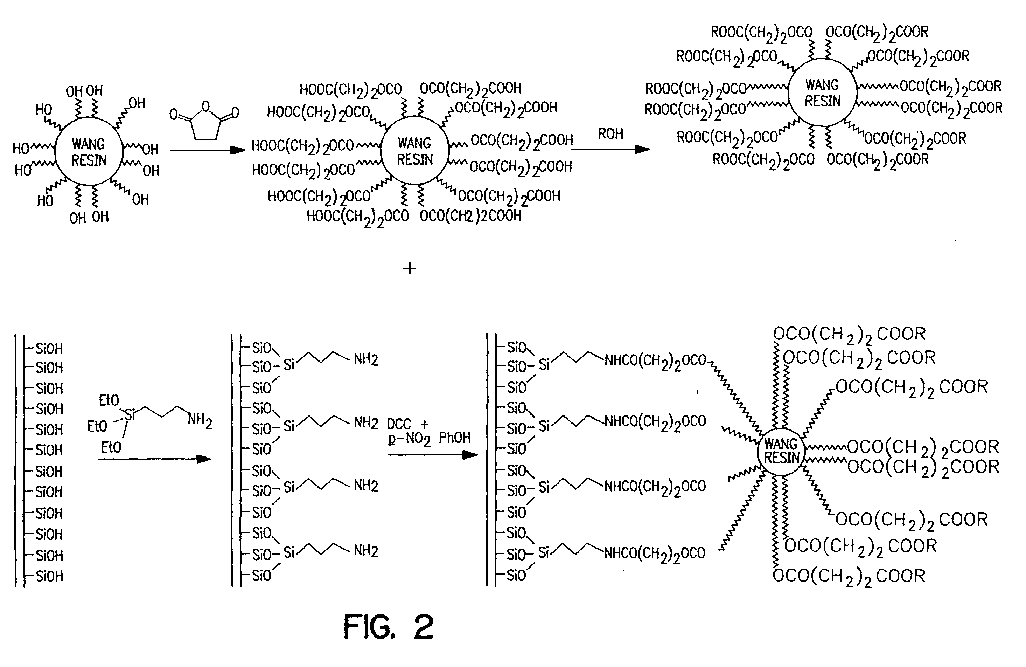

Figure 2 is a schematic showing the covalent attachment of Wang resin beads (p-benzyloxybenzyl alcohol copolystyrene-divinyl benzene (DVB) resin) to a solid support as described in Example 1.

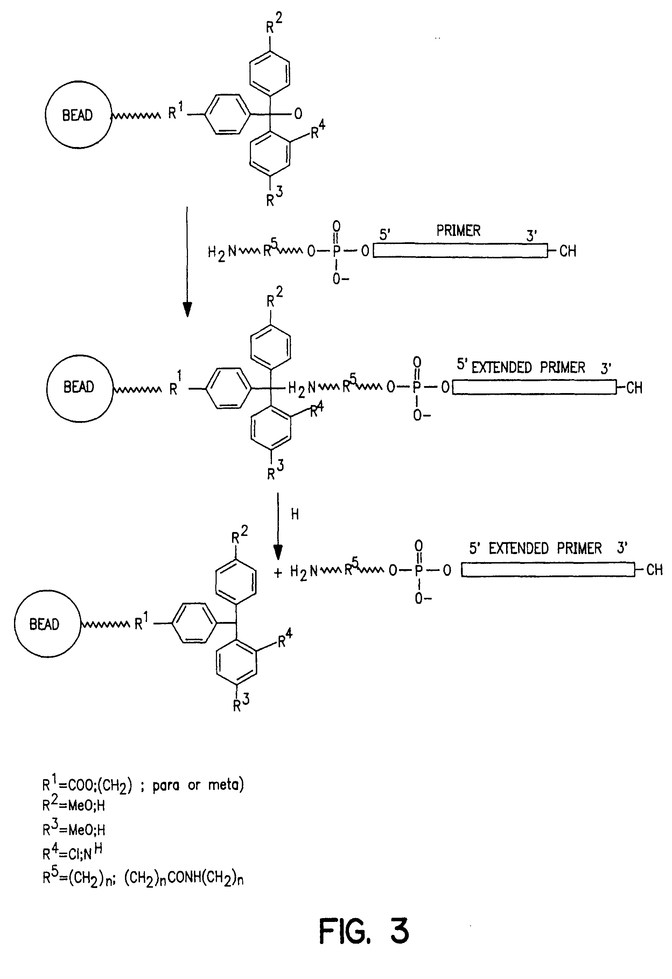

Figure 3 is a schematic representation of nucleic acid immobilization via covalent bifunctional trityl linkers as described in Example 2.

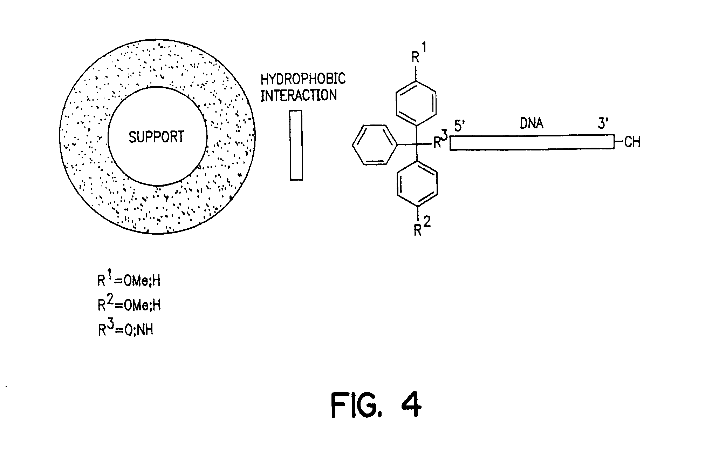

Figure 4 is a schematic representation of nucleic acid immobilization via hydrophobic trityl linkers as described in Example 3.

Figure 5 shows a MALDI-TOF mass spectrum of a supernatant of the matrix treated Dynabeads containing bound oligo (5' iminobiotin-TGCACCTGACTC, SEQ. ID. No. 1). An internal standard (CTGTGGTCGTGC, SEQ. ID. No. 2) was included in the matrix.

Figure 6 shows a MALDI-TOF (matrix-assisted laser desorption/ionization (MALDI)-time-of-flight (TOF)) mass spectrum of a supernatant of biotin treated Dynabeads containing bound oligo (5' iminobiotin-TGCACCTGACTC, SEQ. ID. No. 1). An internal standard (CTGTGGTCGTGC, SEQ. ID. No. 2) was included in the matrix.

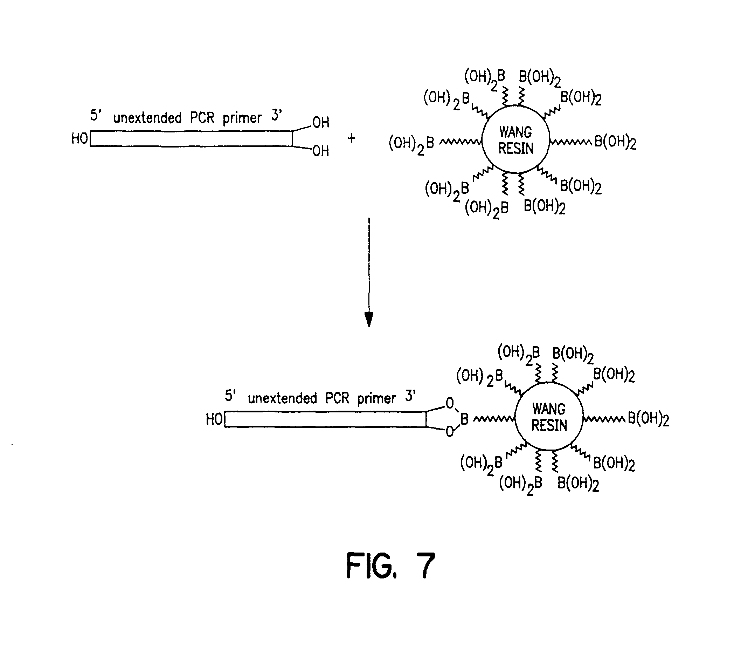

Figure 7 schematically depicts conjugation of an unextended primer to a bead via reaction of a 2', 3'-diol on the primer with boronic acid functionalized beads.

Figure 8 schematically depicts a pin tool apparatus.

Figure 9 depicts various pin conformations. Figures 9A shows a solid pin with a straight head. Figure 9B shows a solid pin with a concave head. Figure 9C shows a solid pin with a truncated pyramidal head. Figure 9D shows a pin with a concave head and a hollowed center (through which can be inserted an optical fibre). Figure 9E shows a pin with a truncated pyramidal head and a hollowed center.

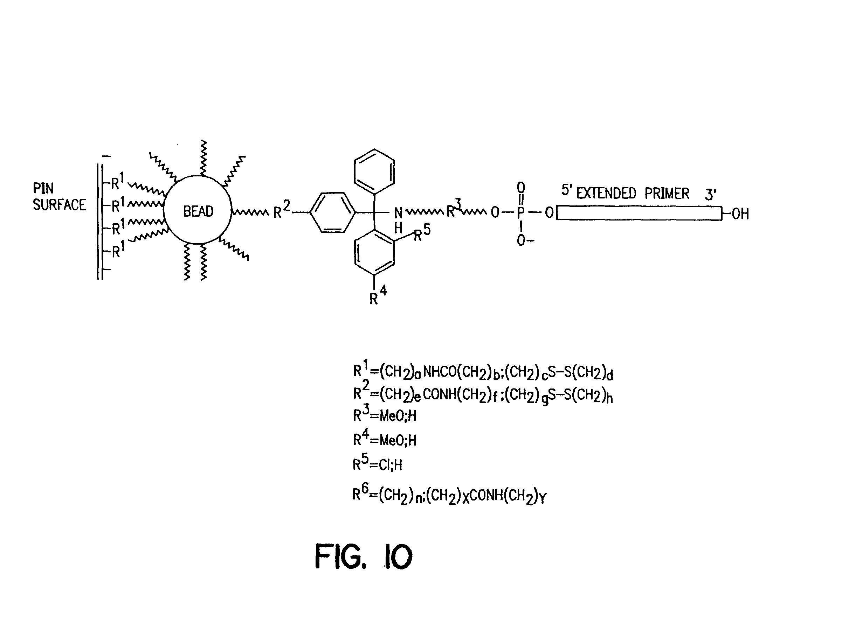

Figure 10 is a schematic representation of the conjugation of beads (activated carboxyl) to pins (amino-functionalized) via amide bonds, and attachment of DNA (via an acid-cleavable linker) to beads. A disulfide linker conjugating the beads to the pins and a thioether conjugation between the bead and the trityl group permits selective cleavage of the beads (with DNA still attached) from the pin surface.

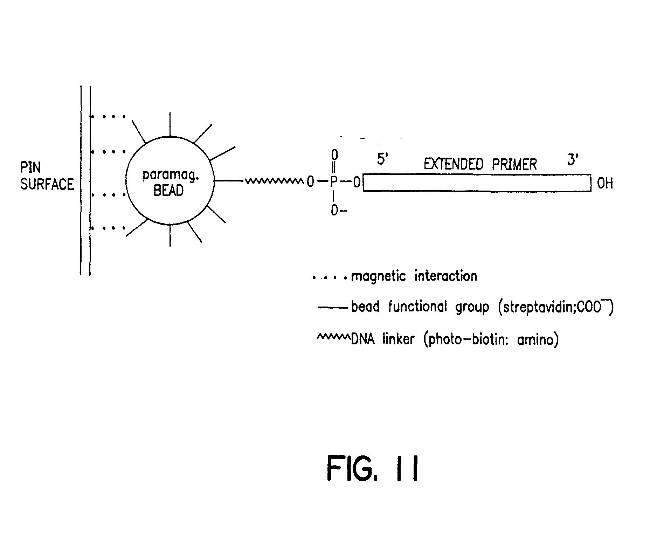

Figure 11 is a schematic representation of paramagnetic beads functionalized with streptavidin to pins via a magnetic interaction and attachment of DNA (via a linker (e.g. modified biotin or photocleavable biotin) to allow selective cleavage of the DNA from the beads.

Figures 12 A-C schematically represent a pin tool apparatus and mount, each separately and a cross section of the mount and tool installed.

Figure 13 is a schematic representation of mass spectrometry geometries for the pin tools shown in Figure 9 A-E.

Figure 14 schematically depicts a pin tool onto which a voltage is applied. When an electrical field is applied, nucleic acids are attracted to the anode. This system purifies nucleic acids, since uncharged molecules would remain in solution, while positively charged molecules are attracted towards the cathode.

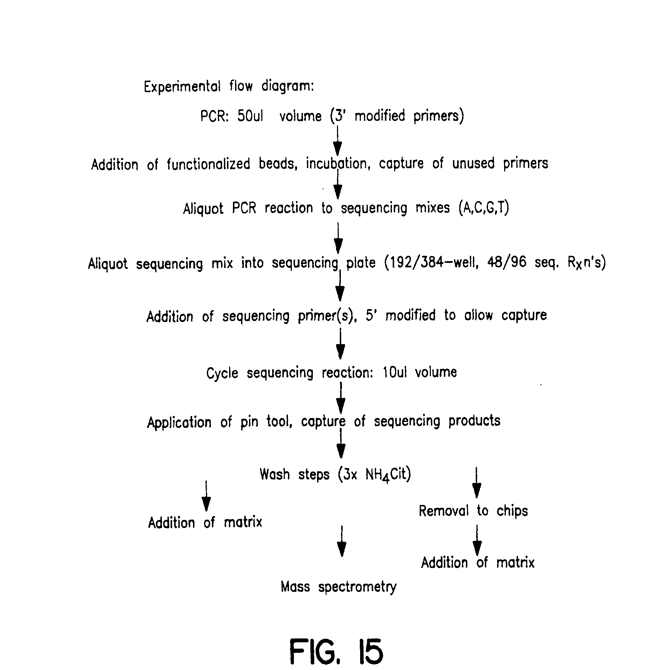

Figure 15 shows a flow chart of the steps involved in sequencing by mass spectrometry using post-biology capture.

### DETAILED DESCRIPTION OF THE INVENTION

### A. Definitions

Unless defined otherwise, all technical and scientific terms used herein have the same meaning as is commonly understood by one of skill in the art to which this invention belongs. All patents and publications referred to herein are, unless noted otherwise, incorporated by reference in their entirety. In the event a definition in this section is not consistent with definitions elsewhere, the definition set forth in this section will control.

As used herein, a molecule refers to any molecule or compound that is linked to the bead. Typically such molecules are macromolecules or components or precursors thereof, such as peptides, proteins, small organics, oligonucleotides or monomeric units of the peptides, organics, nucleic acids and other macromolecules. A monomeric unit refers to one of the constituents from which the resulting compound is built. Thus, monomeric units include, nucleotides, amino acids, and pharmacophores from which small organic molecules are synthesized.

As used herein, macromolecule refers to any molecule having a molecular weight from the hundreds up to the millions. Macromolecules include peptides, proteins, nucleotides, nucleic acids, and other such molecules that are generally synthesized by biological organisms, but can be prepared synthetically or using recombinant molecular biology methods.

As used herein, a biological particle refers to a virus, such as a viral vector or viral capsid with or without packaged nucleic acid, phage, including a phage vector or phage capsid, with or without encapsulated nucleotide acid, a single cell, including eukaryotic and prokaryotic cells or fragments thereof, a liposome or micellar agent or other packaging particle, and other such biological materials. For purposes herein, biological particles include molecules that are not typically considered macromolecules because they are not generally synthesized, but are derived from cells and viruses.

As used herein, the term "nucleic acid" refers to single-stranded and/or double-stranded polynucleotides such as deoxyribonucleic acid (DNA), and ribonucleic acid (RNA) as well as analogs or derivatives of either RNA or DNA. Also included in the term "nucleic acid" are analogs of nucleic acids such as peptide nucleic acid (PNA), phosphorothioate DNA, and other such analogs and derivatives.

As used herein, the term "conjugated" refers stable attachment, preferably ionic or covalent attachment. Among preferred conjugation means are: streptavidin- or avidin- to biotin interaction; hydrophobic interaction; magnetic interaction (e.g., using functionalized magnetic beads, such as DYNABEADS, which are streptavidin-coated magnetic beads sold by Dynal, Inc. Great Neck, NY and Oslo Norway); polar interactions, such as "wetting" associations between two polar surfaces or between oligo/polyethylene glycol; formation of a covalent bond, such as an amide bond, disulfide bond, thioether bond, or via crosslinking agents; and via an acid-labile or photocleavable linker.

### B. Preparation of solid supports with macromolecules or biological particles immobilized via linkage to beads

Provided herein are solid supports with linked macromolecules or biological particles that are linked to the supports via beads. The beads are appropriately functionalized for such linkage and are of any suitable shape. For exemplification herein, nucleic acids are described. It, however, is understood, the methods and solid supports with linked beads can be used for immobilization other macromolecules and for biological particles, and any other molecules suitable for immobilization on such supports.

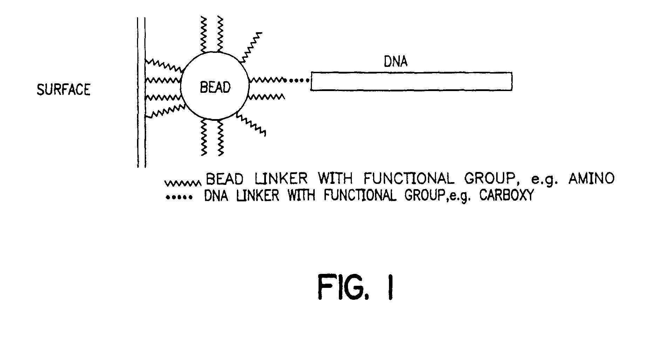

In preferred embodiments, the beads are functionalized for the immobilization of nucleic acids and are stably associated with a solid support. Figure 1 depicts a bead conjugated to a solid support through one or more covalent or non-covalent bonds. Nucleic acids can be immobilized on the functionalized bead before, during or after the bead is conjugated to the solid support.

Preferred nucleic acids for use herein are derivatized to contain at least one reactive moiety. Preferably the reactive moiety is at the 3' or 5' end. Alternatively, a nucleic acid can be synthesized with a modified base. In addition, modification of the sugar moiety of a nucleotide at positions other than the 3' and 5' position through conventional methods is contemplated. Also, nucleic acid bases can be modified, e.g., by using N7- or N9- deazapurine nucleosides or by modification of C-5 of dT with a linker arm (see, e.g., Eckstein, ed., "Oligonucleotides and Analogues: A Practical Approach," IRL Press (1991)). Alternatively, backbone-modified nucleic acids (e.g., phosphoroamidate DNA) can be used so that a reactive group can be attached to the nitrogen center provided by the modified phosphate backbone.

In preferred embodiments, modification of a nucleic acid, e.g., as described above, does not substantially impair the ability of the nucleic acid or nucleic acid sequence to hybridize to its complement. Thus, any modification should preferably avoid substantially modifying the functionalities of the nucleic acid which are responsible for Watson-Crick base pairing. The nucleic acid can be modified such that a non-terminal reactive group is present, and the nucleic acid, when immobilized to the support, is capable of self-complementary base pairing to form a "hairpin" structure having a duplex region.

### Supports

Supports may be of any form including beads, (silica gel, controlled pore glass, magnetic beads, Dynabeads, Wang resin; Merrifield resin, Sephadex/Sepharose beads, cellulose beads, etc.), capillaries, flat supports such as glass fiber filters, glass surfaces, metal surfaces (steel, gold silver, aluminum, silicon and copper), plastic materials including multiwell plates or membranes (e.g., formed of polyethylene, polypropylene, polyamide, polyvinylidenedifluoride), wafers, combs, pins or needles (e.g., arrays of pins suitable for combinatorial synthesis or analysis) or beads in an array of pits or nanoliter wells of flat surfaces such as wafers (e.g. silicon wafers), wafers with pits with or without filter bottoms.

Thus, combinatorial libraries of immobilized nucleic acids or other macromolecules or biological particles, bound to beads, which are further bound to a solid support as described above, are provided.

### Beads

Appropriate "beads" for use herein include any three dimensional structure that can be conjugated to a solid support and provides an increased surface area for immobilization of biological particles and macromolecules, such as DNA and RNA. Preferably the bead is of a size in the range of about 1 to about 100 *µ*m in diameter. For purposes herein, a bead can be made of virtually any insoluble or solid material. For example, the bead can be made of silica gel, glass (e.g. controlled-pore glass (CPG)), nylon, Wang resin, Merrifield resin, Sephadex®, Sepharose® , cellulose, magnetic beads, Dynabeads, a metal surface (e.g. steel, gold, silver, aluminum, silicon and copper), a plastic material (e.g., polyethylene, polypropylene, polyamide, polyester, polyvinylidenedifluoride (PVDF)) and the like. Beads can be swellable, e.g., polymeric beads such as Wang resin, or non-swellable (e.g., CPG).

### Conjugation

The biological particles and macromolecules, such as nucleic acid molecules can be attached directly to the beads via a linker. Conjugation can be through any suitable means, particularly covalent or non-covalent attachment. For example, in one embodiment for conjugating nucleic acids to beads, the conjugating means introduces a variable spacer between the beads and the nucleic acids. In another embodiment, the conjugation is directly cleavable, such as a photocleavable linkage (e.g. streptavidin- or avidin- to biotin interaction can be cleaved by a laser, for example for mass spectrometry) or indirectly via photocleavable linker (see, e.g., U.S. Patent No. 5,643,722) or acid labile linker, heat sensitive linker, enzymatically cleavable linker or other such linker.

Similarly the bead is conjugated to the solid support by any suitable means, including those discussed herein for attachment of nucleic acids to beads. Thus, any of the conjugation methods and means discussed below with reference to conjugation of nucleic acids to beads can be applied for conjugation of beads to the solid support. In addition, it is understood that nucleic acids are exemplary of the molecules that can be conjugated to beads.

### Conjugation via Linkers

Appropriate cross-linking agents for use for conjugating molecules to supports and beads and/or the beads to the supports include a variety of agents that are capable of reacting with a functional group present on a surface of the bead, insoluble support and or molecule, such as nucleic acid, and with a functional group present in the molecule, such as the nucleic acid and/or bead, respectively. Reagents capable of such reactivity include homo- and hetero-bifunctional reagents, many of which are known in the art. Heterobifunctional reagents are preferred. A preferred bifunctional cross-linking agent is N-succinimidyl(4-iodoacetyl) aminobenzoate (SIAB). Oher crosslinking agents, including, without limitation, dimaleimide, dithio-bis-nitrobenzoic acid (DTNB), N-succinimidyl-S-acetyl-thioacetate (SATA), N-succinimidyl-3-(2-pyridyldithio) propionate (SPDP), succinimidyl 4-(N-maleimidomethyl)-cyclohexane-1-carboxylate (SMCC) and 6-hydrazinonicotimide (HYNIC) may also be used herein.

In certain embodiments, the cross-linking agent can be selected to provide a selectively cleavable bond when the nucleic acid molecule is immobilized on the insoluble support. For example, a photolabile crosslinker such as 3-amino-(2-nitrophenyl)propionic acid (Brown et al. (1995) Molecular Diversity 4-12; and Rothschild et al. (1996) Nucl. Acids Res. 24:351-66; see, also, U.S. Patent No. 5,643,722) can be employed to provide a means for cleaving the nucleic acid from the beads or insoluble (e.g., solid) support, if desired. Other cross-linking reagents are well-known (see, e.g., Wong (1991) "Chemistry of Protein Conjugation and Cross-Linking," CRC Press; and Hermanson (1995) "Bioconjugate Techniques," Academic Press).

In another preferred embodiment, a covalent amide bond is formed between a bead and a insoluble support by reacting a carboxyl-functionalized bead with an amino-functionalized solid support (e.g., as described in Example 1, below, by reacting a carboxyl-functionalized Wang resin with an amino-functionalized silicon surface).

Alternatively, a carboxyl-functionalized support can be reacted with an amino-functionalized bead, which take advantage of an acid-cleavable bifunctional trityl protection scheme employed for nucleic acid attachment. The bifunctional trityl linker can also be attached to the 4-nitrophenyl active ester on a resin (e.g., Wang resin) via an amino group as well as from a carboxy group via an amino resin. In the bifunctional trityl approach, the beads may require treatment with a volatile acid e.g., formic acid, trifluoracetic acid, etc.) to ensure that the nucleic acid is cleaved and can be removed. In which case, the nucleic acid may be deposited as a beadless patch at the bottom of a well in the solid support or on the flat surface of the solid support. After addition of matrix solution, the nucleic acid can then be desorbed, for example into a mass spectrometer.

Hydrophobic trityl linkers can also be exploited as acid-labile linkers by using a volatile acid or an appropriate matrix solution (e.g. a matrix solution containing, for example, 3-hydroxypicolinic acid (3-HPA) to cleave the aminolink trityl group from the nucleic acid molecule). Also, the acid lability can be changed. For example, trityl, monomethoxy, dimethoxy- or trimethoxytrityl can be changed to the appropriate p-substituted and even more acid labile tritylamine derivatives of the nucleic acids (i.e. trityl ether and tritylamine bonds to the nucleic acid can be made). Therefore, the nucleic acid may be removed from the hydrophobic linker, for example, by disrupting the hydrophobic attraction or by cleaving tritylether or tritylamine bonds under acidic or the usual mass spectrometry conditions (e.g. wherein the matrix, such as 3-HPA acts as an acid).

In a particularly preferred embodiment the bead is conjugated to the solid support and/or the nucleic acid is conjugated to the bead using an acid-labile bond. For example, use of a trityl linker, as further described in the following Examples 2 and 3, can provide a covalent or hydrophobic conjugation. Regardless of the nature of the conjugation, the trityl group is readily cleaved in acidic conditions.

In certain embodiments, orthogonally-cleavable linkers can be used for binding the bead to the solid support, and for binding the nucleic acid to the bead. Thus, a bead can be selectively cleaved from the surface without cleaving the nucleic acid from the bead, while the nucleic acid is cleaved from the bead at a later stage. For example, a disulfide linker (which can be cleaved, using, e.g., DTT) could be employed to bind the bead to the solid surface, and a bead-nucleic acid linker involving an acid-cleavable bifunctional trityl group could be used to immobilize a nucleic acid to the bead. Alternatively the linkage of the nucleic acid could be cleaved while the linkage of the bead to the support remains intact.

### Non-covalent linkage

As pointed out above, the bead can also be associated with the solid support by non-covalent interactions. For example, a magnetic bead (e.g., a bead capable of being magnetized, e.g., a ferromagnetic bead) can be attracted to a magnetic solid support, and can be released from the support by removal of the magnetic field. Alternatively, the bead can be provided with an ionic or hydrophobic moiety, which can associate with, respectively, an ionic or hydrophobic moiety of the solid support. Also, a bead can be provided with a member of a specific binding pair, and become immobilized to a solid support provided with a complementary binding moiety. For example, a bead coated with avidin or streptavidin can be bound to a surface coated with biotin or derivatives of biotin such as imino-biotin.

It will be appreciated that the binding members can be reversed, e.g., a biotin-coated bead can bind to a streptavidin-coated solid support. Other specific binding pairs contemplated for use herein include but are not limited to hormone-receptor, enzyme-substrate, nucleic acid-complementary nucleic acid, antibody-antigen and other such pairs know to those of skill in the art.

Examples of preferred binding pairs or linker/interactions is shown in the following Table 1:

**TABLE 1**

| **LINKER/INTERACTION** | **EXAMPLES** |
|---|---|
| streptavidin-biotin^{a,c}/photolabile biotin^{b} | biotinylated pin, avidin beads, photolabile biotin DNA |
| hydrophobic^{a} | C18-coated pin, tritylated DNA |
| magnetic^{a} | electromagnetic pin, steptavidin magnetic beads (e.g., DYNABEADS), biotin DNA |
| acid-labile linker^{b} | glass pin, bifunctional trityl-linked DNA |
| amide bond(s)^{c} | silicon wafer, Wang resin, amino-linked DNA |
| disulfide bond^{a} | silicon wafer, beads are bound on the flat surface forming arrays or in arrays of nanoliter wells, thiol beads, thiolated DNA |
| photocleavable bond/linker | biotinylated pin/wafer, avidin beads, photolabile biotin DNA |
| thioether bond^{c} | silicon wafer, beads are bound on the flat surface forming arrays or in arrays of nanoliter wells, thiolated DNA |

| | |
|---|---|
| ^{a}These interactions are reversible. | |
| ^{b}These non-reversible interactions are rapidly cleaved. | |
| ^{c}Unless cleavable-linkers are incorporated at some point in the scheme, only the complement of the solid-bound DNA can be analyzed in these schemes. | |

In a particularly preferred embodiment the bead is conjugated to the solid support and/or the nucleic acid is conjugated to the bead using an acid-labile bond. For example, use of a trityl linker, as further described in the following Examples 2 and 3, can provide a covalent or hydrophobic conjugation. Regardless of the nature of the conjugation, the trityl group is readily cleaved in acidic conditions. Photocleavable linkers are also contemplated herein.

In certain embodiments, orthogonally-cleavable linkers can be used for binding the bead to the solid support, and for binding the nucleic acid to the bead. Thus, a bead can be selectively cleaved from the surface without cleaving the nucleic acid from the bead, while the nucleic acid is cleaved from the bead at a later stage. For example, a disulfide linker (which can be cleaved, using, e.g., DTT) could be employed to bind the bead to the solid surface, and a bead-nucleic acid linker involving an acid-cleavable bifunctional trityl group could be used to immobilize a nucleic acid to the bead. Alternatively the linkage of the nucleic acid could be cleaved while the linkage of the bead to the support remains intact.

### Conjugation of beads to solid supports

Beads can be attached to solid supports by the methods and linkages and conjugation means discussed above. Also, a bead can be bound to a solid support through a linking group which can be selected to have a length and a chemical nature such that high-density binding of beads to the solid support, and/or high-density binding of nucleic acid to the beads, is promoted. Such a linking group would have a "tree-like" structure in providing a multiplicity of functional groups per attachment site on a solid support such as polylysine, polyglutamic acid, pentaerythrole and tris-hydroxy-aminomethane.

In certain embodiments, beads can be cross-linked to other beads, e.g., by use of homobifunctional crosslinking reagents. Cross-linked beads can provide additional mechanical strength compared to non-crosslinked beads.

### Applications

The solid supports with beads and immobilized molecules can be used in any application for which solid supports with immobilized molecules are used. For example, the methods and compositions described herein, can be used to isolate (purify) target nucleic acids from biological samples (reactions). For example, the compositions and methods can be used to isolate particular nucleic acids, which are generated by cloning (Sambrook et al., Molecular Cloning : A Laboratory Manual, Cold Spring Harbor Laboratory Press, 1989), polymerase chain reaction (PCR) (C.R. Newton and A. Graham, PCR, BIOS Publishers, 1994), ligase chain reaction (LCR) (see, e.g., Wiedmann et al. (1994) PCR Methods Appl. 3:57-64; Barany (1991) Proc. Natl. Acad. Sci. U.S.A. 88:189-93), strand displacement amplification (SDA) (see, e.g., Walker et al. (1994) Nucl. Acids Res. 22:2670-77; European Patent Publication Number 0 684 315 entitled "Strand Displacement Amplification Using Thermophilic Enzymes") and variations such as reverse transcriptase (RT)-PCR (Higuchi et al. (1993) Big/Technology 11:1026-1030), allele-specific amplification (ASA), cycle sequencing and transcription based processes.

Further the methods and compositions can be used to isolate or transfer particular nucleic acids during the performance of a particular reaction. For example, an amplification reaction, such as a PCR reaction, can be performed to 'master' mix without addition of the dideoxynucleotides (d/ddNTPs) or sequencing primers. Aliquots can be isolated via a conjugation means described herein and transferred, for example to a sequencing plate, where d/ddNTPs and primers can then be added to perform a sequencing reaction. Alternatively, the PCR can be split between A, C, G, and T master mixes. Aliquots can then be transferred to a sequencing plate and sequencing primers added.

For example, 0.4-0.5 pmol of PCR product can be used in a cycle-sequencing reaction using standard conditions, allowing each PCR to be used for 10 sequencing reactions (10 x A, C, G, and T). The sequencing reactions can be carried out in a volume of 10 *µ*l containing 5-6 pmol of 5'-labeled sequencing primer in a standard 384 microwell plate allowing up to 96 sequencing reactions (3360 bases at 35 bases per reaction). Alternatively, a 192 microwell plate approximately 5 x 5 cm in a 12 x 16 format can be used. This format allows up to 48 sequencing reactions to be carried out per well, resulting in 1680 bases per plate (at 35 bases per reaction). The format of the sequencing plate will determine the dimensions of the transfer agent (e.g., a pin tool).

### Pin tools

Pin tools include those known to the skilled artisan, those depicted herein and also those, discussed below, that are the subject of copending U.S. application Serial Nos. 08/786,988 and 08/787,639.

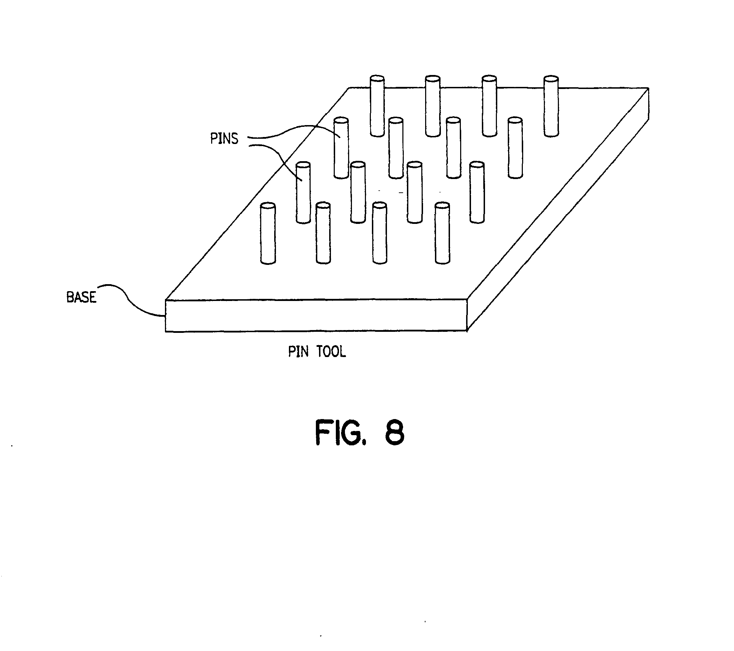

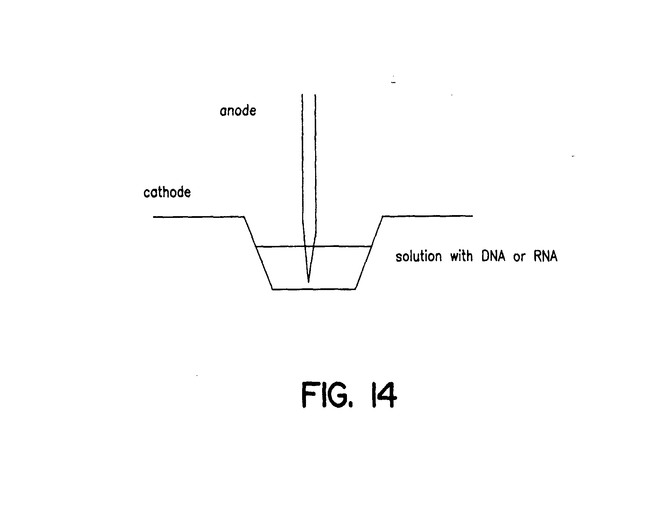

As exemplified herein, a pin tool in an array, a 4 X 4 array (Fig. 8) is depicted, can be applied to the wells of the sequencing plate and the sequencing products captured on functionalized beads as described herein, which are attached to the tips of the pins (> = 1 pmol capacity). During the capture/incubation step, the pins can be kept in motion (vertical, 1-2 mm travel) to mix the sequencing reaction and increase the efficiency of the capture. Alternatively, the nucleic acid can be directly captured onto the pin tool, for example, a linking functionality on the pin tool can immobilize the nucleic acid upon contact. Further, immobilization can result from application to the pin tool of an electrical field, as shown in Figure 14. When a voltage is applied to the pin tool, the nucleic acids are attracted to the anode. This system also purifies nucleic acids, since uncharged molecules remain in solution and positively charged molecules are attracted to the cathode. For more specificity, the pin tool (with or without voltage), can be modified to contain a partially or fully single stranded oligonucleotide (e.g., about 5-12 base pairs). Only complementary nucleic acid sequences (e.g. in solution) are then specifically conjugated to the pins.

In yet a further embodiment, a PCR primer can be conjugated to the tip of a pin tool. PCR can be performed with the solid phase (pin tool)-bound primer and a primer in solution, so that the PCR product becomes attached to the pin tool. The pin tool with the amplification product can then be removed from the reaction and analyzed (e.g., by mass spectrometry).

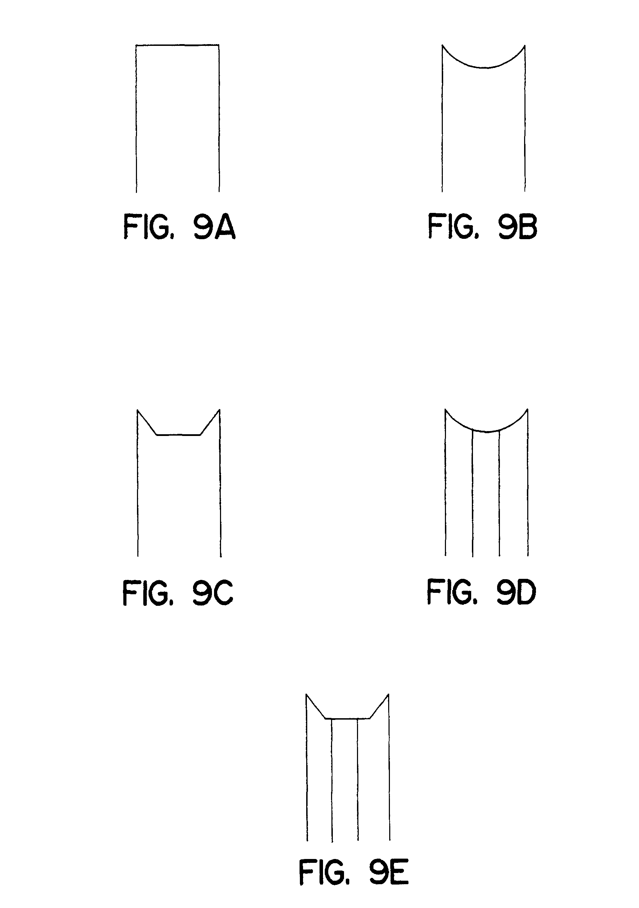

Examples of different pin conformations are shown in Figure 9. For example, Figures 9a, 9b and 9c show a solid pin configuration. Figures 9d and 9e show pins with a channel or hole through the center, for example to accommodate an optic fiber for mass spectrometer detection. The pin can have a flat tip or any of a number of configurations, including nanowell, concave, convex, truncated conic or truncated pyramidal (e.g. size 4-800*µ* across x 100*µ* depth). In a preferred embodiment, the individual pins, which can be any desired size, are preferably up to about 10 mm in length, and more preferably are about 5 mm in length and about 1 mm in diameter. The pins and mounting plate can be made of polystyrene (e.g. one-piece injection molded). Polystyrene is an ideal material to be functionalized and can be molded with very high tolerances. The pins in a pin tool apparatus may be collapsible (e.g., controlled by a scissor-like mechanism), so that pins may be brought into closer proximity, reducing the overall size.

Captured nucleic acids can be analyzed by any of a variety of means including, for example, spectrometric techniques such as UV/VIS, IR, fluorescence, chemiluminescence, or NMR spectroscopy, mass spectrometry, or other methods known in the art, or combinations thereof. Preferred mass spectrometer formats include ionization (I) techniques, such as matrix assisted laser desorption (MALDI), continuous or pulsed electrospray (ESI) and related methods (e.g. lonspray or Thermospray), or massive cluster impact (MCI); these ion sources can be matched with detection formats including linear or non-linear reflection time-of-flight (TOF), single or multiple quadrupole, single or multiple magnetic sector, Fourier Transform ion cyclotron resonance (FTICR), ion trap, and combinations thereof (e.g., ion-trap/time-of-flight). For ionization, numerous matrix/wavelength combinations (MALDI) or solvent combinations (ESI) can be employed.

If conditions preclude direct analysis of captured DNA, then the DNA can be released and/or transferred. It may be important that the advantages of sample concentration are not lost at this stage. Preferably, the sample should be removed from the surface in as little a volume of eluent as possible, and without any loss of sample. Another alternative is to remove the beads (+ sample) from the surface, where relevant, and measure the sample directly from the beads.

For example, for detection by mass spectrometry, the pin tool can be withdrawn and washed several times, for example in ammonium citrate to condition the sample before addition of matrix. For example, the pins can simply be dipped into matrix solution. The concentration of matrix can then be adjusted such that matrix solution only adheres to the very tip of the pin. Alternatively, the pin tool can be inverted and the matrix solution sprayed onto the tip of each pin by a microdrop device. Further, the products can be cleaved from the pins, for example into a nanowell on a chip, prior to addition of matrix.

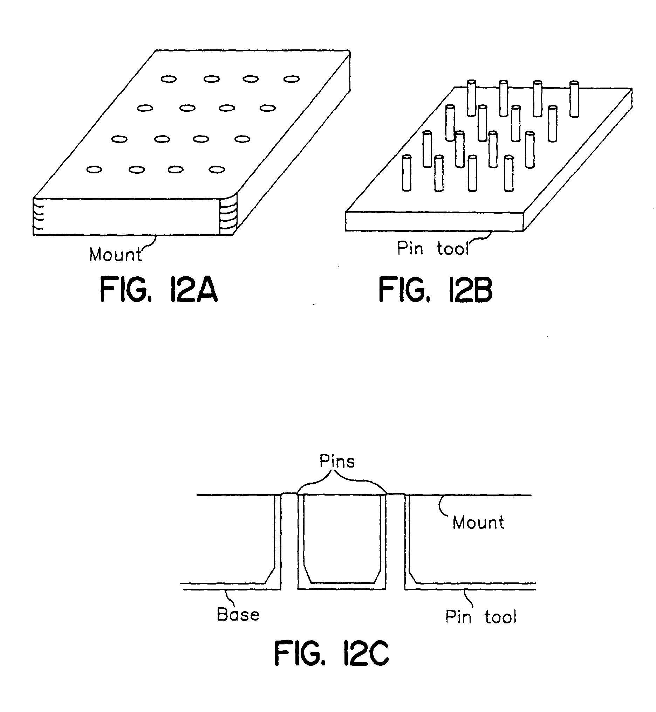

For analysis directly from the pins, a stainless steel 'mask' probe can be fitted over the pins in one scheme (Fig. 12) which can then be installed in the mass spectrometer.

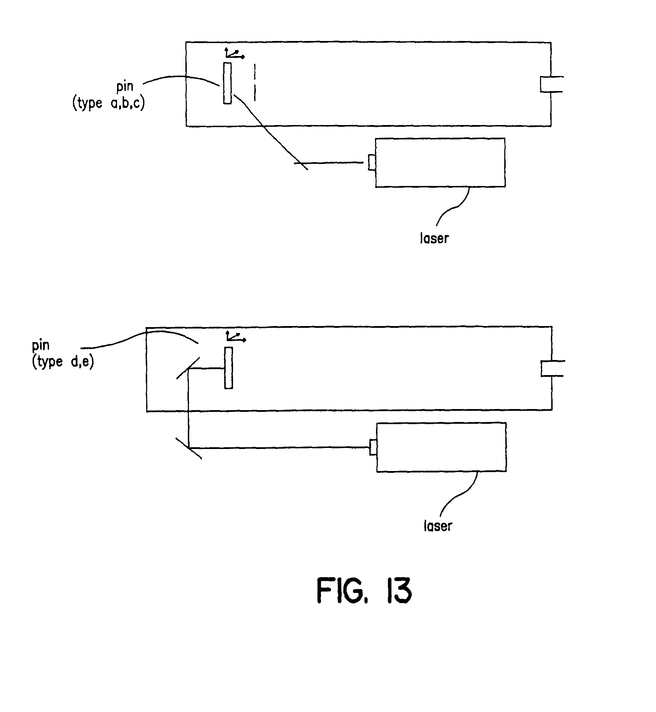

Two mass spectrometer geometries for accommodating the pin tool apparatus (see Figure 9) are proposed in Figure 13. The first accommodates solid pins. In effect, the laser ablates a layer of material from the surface of the crystals, the resultant ions being accelerated and focused through the ion optics. The second geometry accommodates fibre optic pins in which the samples are lasered from behind. In effect, the laser is focused onto the pin tool back plate and into a short optical fibre (about 100 *µ*m in diameter and about 7 mm length to include thickness of the back plate). This geometry requires the volatilized sample to go through the depth of the matrix/bead mix, slowing and cooling down the ions resulting in a type of delayed extraction which should actually increase the resolution of the analysis.

The probe through which the pins are fitted can also be of various geometries. For example, a large probe with multiple holes, one for each pin, fitted over the pin tool. The entire assembly is translated in the X-Y axes in the mass spectrometer. Alternatively, as a fixed probe with a single hole, which is large enough to give an adequate electric field, but small enough to fit between the pins. The pin tool is then translated in all three axes with each pin being introduced through the hole for sequential analyses. This format is more suitable for the higher density pin tool (i.e. based on a 384 well or higher density microplate format). The two probes described above, are suitable for the two mass spectrometer geometries described above.

Figure 14 schematically depicts the steps involved in mass spectrometry sequencing by post biology capture as described above.

### Preparation of arrays that include beads in a flat surface or in wells

The methods provided herein are useful for providing spatiallyaddressable arrays of nucleic acids immobilized on beads, which are further attached to solid supports. Such spatially addressable or preaddressable arrays are useful in a variety of processes (e.g., SBH, quality control, and DNA sequencing diagnostics). The tools described in the copending applications U.S. application Serial Nos. 08/786,988 and 08/787,639, therein are serial and parallel dispensing tools that can be employed to generate multi-element arrays of sample material on a substrate surface. The substrates surfaces can be flat, with beads or geometrically altered to include wells, preferably containing beads, of receiving material. In one embodiment, a tool that allows the parallel development of a sample array is provided. To this end, the tool is an assembly of vesicle elements, or pins, where each of the pins can include a narrow interior chamber suitable for holding nanoliter volumes of fluid. Each of the pins fits inside a housing that has an interior chamber. The interior housing can be connected to a pressure source that will control the pressure within the interior housing chamber to regulate the flow of fluid through the interior chamber of the pins. This allows for the controlled dispensing of defined volumes of fluid from the vesicles.

In an alternative embodiment, the tool includes a jet assembly that can include a capillary pin having an interior chamber, and a transducer element mounted to the pin and capable of driving fluid through the interior chamber of the pin to eject fluid from the pin. In this way, the tool can dispense a spot of fluid to a substrate surface by spraying the fluid from the pin. Alternatively, the transducer can cause a drop of fluid to extend from the capillary so that fluid can be passed to the substrate by contacting the drop to the surface of the substrate. Further, the tool can form an array of sample material by dispensing sample material in a series of steps, while moving the pin to different locations above the substrate surface to form the sample array. In a further embodiment, the tool then passes prepared sample arrays to a plate assembly that disposes the sample arrays for analysis by mass spectrometry. A mass spectrometer is provided that generates a set of spectra signal that is indicative of the composition of the sample material under analysis.

In particular, the pin tool includes a housing having a plurality of sides and a bottom portion having formed therein a plurality of apertures, the walls and bottom portion of the housing defining an interior volume; one or more fluid transmitting vesicles, or pins, mounted within the apertures, having a nanovolume sized fluid holding chamber for holding nanovolumes of fluid, the fluid holding chamber being disposed in fluid communication with the interior volume of the housing, and a dispensing element that is in communication with the interior volume of the housing for selectively dispensing nanovolumes of fluid form the nanovolume sized fluid transmitting vesicles when the fluid is loaded with the fluid holding chambers of the vesicles. This allows the dispensing element to dispense nanovolumes of the fluid onto the surface of the substrate when the apparatus is disposed over and in registration with the substrate.

In one embodiment, the fluid transmitting vesicle has an open proximal end and a distal tip portion that extends beyond the housing bottom portion when mounted within the apertures. In this way the open proximal end can dispose the fluid holding chamber in fluid communication with the interior volume when mounted with the apertures. Optionally, the plurality of fluid transmitting vesicles are removably and replaceably mounted within the apertures of the housing, or alternatively can include a glue seal for fixedly mounting the vesicles within the housing.

In another embodiment, the fluid holding chamber includes a narrow bore dimensionally adapted for being filled with the fluid through capillary action, and can be sized to fill substantially completely with the fluid through capillary action. The plurality of fluid transmitting vesicles comprise an array of fluid delivering needles, which can be formed of metal, glass, silica, polymeric material, or any other suitable material, and, thus, as described herein, can also serve as a solid support.

In one embodiment the housing can include a top portion, and mechanical biasing elements for mechanically biasing the plurality of fluid transmitting vesicles into sealing contact with the housing bottom portion. In one particular embodiment, each fluid transmitting vesicle has a proximal end portion that includes a flange, and further includes a seal element disposed between the flange and an inner surface of the housing bottom portion for forming a seal between the interior volume and an external environment. The biasing elements can be mechanical and can include a plurality of spring elements each of which are coupled at one end to the proximal end of each the plurality of fluid transmitting vesicles, and at another end to an inner surface of the housing top portion. The springs can apply a mechanical biasing force to the vesicle proximal end to form the seal.

In a further embodiment, the housing further includes a top portion, and securing element for securing the housing top portion to the housing bottom portion. The securing element can comprise a plurality of fastener-receiving apertures formed within one of the top and bottom portions of the housing, and a plurality of fasteners for mounting within the apertures for securing together the housing top and bottom portions.

In one embodiment the dispensing element includes a pressure source fluidly coupled to the interior volume of the housing for disposing the interior volume at a selected pressure condition. Moreover, in an embodiment wherein the fluid transmitting vesicles are filled through capillary action, the dispensing element can include a pressure controller than can vary the pressure source to dispose the interior volume of the housing at varying pressure conditions. This allows the controller varying element to dispose the interior volume at a selected pressure condition sufficient to offset the capillary action to fill the fluid holding chamber of each vesicle to a predetermined height corresponding to a predetermined fluid amount. Additionally, the controller can further include a fluid selection element for selectively discharging a selected nanovolume fluid amount from the chamber of each the vesicle. In one particular embodiment, the a pressure controller that operates under the controller of a computer program operating on a data processing system to provide variable control over the pressure applied to the interior chamber of the housing is provided.

The fluid transmitting vesicle can have a proximal end that opens onto the interior volume of the housing, and the fluid holding chamber of the vesicles are sized to substantially completely fill with the fluid through capillary action without forming a meniscus at the proximal open end. Optionally, the apparatus can have plural vesicles, wherein a first portion of the plural vesicles include fluid holding chambers of a first size and a second portion including fluid holding chambers of a second size, whereby plural fluid volumes can be dispensed.

In another embodiment, the tool can include a fluid selection element that has a pressure source coupled to the housing and in communication with the interior volume for disposing the interior volume at a selected pressure condition, and an adjustment element that couples to the pressure source for varying the pressure within the interior volume of the housing to apply a positive pressure in the fluid chamber of each the fluid transmitting vesicle to vary the amount of fluid dispensed therefrom. The selection element and adjustment element can be computer programs operating on a data processing system that directs the operation of a pressure controller connected to the interior chamber.

In a further alternative embodiment, the pin tool apparatus is for dispensing a fluid in chemical or biological procedures into one or more wells of a multi-well substrate. The apparatus can include a housing having a plurality of sides and a bottom portion having formed therein a plurality of apertures, the walls and bottom portion defining an interior volume, a plurality of fluid transmitting vesicles, mounted within the apertures, having a fluid holding chamber disposed in communication with the interior volume of the housing, and a fluid selection and dispensing means in communication with the interior volume of the housing for variably selecting am amount of the fluid loaded within the fluid holding chambers of the vesicles to be dispensed from a single set of the plurality of fluid transmitting vesicles. Accordingly, the dispensing means dispenses a selected amount of the fluid into the wells of the multi-well substrate when the apparatus is disposed over and in registration with the substrate.

In yet another embodiment, the fluid dispensing apparatus for dispensing fluid in chemical or biological procedures into one or more wells of a multi-well substrate, includes a housing having a plurality of sides and top and bottom portions, the bottom portion having formed therein a plurality of apertures, the walls and top and bottom portions of the housing defining an interior volume, a plurality of fluid transmitting vesicles, mounted within the apertures, having a fluid holding chamber sized to hold nanovolumes of the fluid, the fluid holding chamber being disposed in fluid communication with the volume of the housing, and mechanical biasing element for mechanically biasing the plurality of fluid transmitting vesicles into sealing contact with the housing bottom portion.

### Kits

Also provided is a kit for immobilizing nucleic acids on beads, which are further bound. The kit includes an appropriate amount of: i) beads, and ii) the insoluble support, and iii) conjugation means. The kits described herein can also optionally include appropriate buffers; containers for holding the reagents; and/or instructions for use.

The following examples are included for illustrative purposes only and are not intended to limit the scope of the invention.

### EXAMPLE 1

### Attachment of Resin Beads to a Silicon Surface

A silicon surface (e.g. of a silicon wafer) is derivatized with amino groups by treatment with 3-aminopropyltriethoxysilane. Wang resin beads are treated with succinic anhydride to provide carboxyl-functionalized resin beads. The carboxyl-functionalized resin beads are then coupled to the amino-functionalized silicon surface with a coupling reagent (for example, dicyclohexylcarbodiimide (DCC)), in the presence of p-nitrophenol. The resin beads become covalently linked to the silicon surface, and the unreacted carboxyl groups of the resin are converted to the p-nitrophenyl ester (an activated ester suitable for coupling with a nucleic acid).

Alternatively, the carboxyl groups of the Wang resin are transformed to the p-nitrophenyl active esters prior to reacting with the amino-functionalized silicon surface.

Thus, resin beads can be rapidly and conveniently attached to a silicon surface, and can be simultaneously converted to a reactive form suitable for covalent attachment of nucleic acids.

### EXAMPLE 2

### Immobilization of nucleic acids on solid supports via an acid-labile covalent bifunctional trityl linker.

Aminolink DNA was prepared and purified according to standard methods. A portion (10 eq) was evaporated to dryness on a speedvac and suspended in anhydrous DMF/pyridine (9:1; 0.1 ml). To this was added the chlorotrityl chloride resin (1 eq, 1.05 mol/mg loading) and the mixture was shaken for 24 hours. The loading was checked by taking a sample of the resin, detritylating this using 80% AcOH, and measuring the absorbance at 260 nm. Loading was ca. 150 pmol/mg resin.

In 80% acetic acid, the half-life of cleavage was found to be substantially less than 5 minutes-this compares with trityl ether-based approaches of half-lives of 105 and 39 minutes for *para* and *meta* substituted bifunctional dimethoxytrityl linkers respectively. Preliminary results have also indicated that the 3-hydroxy picolinic acid matrix alone is sufficient to cleave the DNA from the chlorotrityl resin during MALDI mass spectrometry.

### EXAMPLE 3

### Immobilization of nucleic acids on solid supports via hydrophobic trityl linker.

The primer contained a 5'-dimethoxytrityl group attached using routine trityl-on DNA synthesis.

C18 beads from an oligo purification cartridge (0.2 mg) placed in a filter tip was washed with acetonitrile, then the solution of DNA (50 ng in 25 I) was flushed through. This was then washed with 5% acetonitrile in ammonium citrate buffer (70 mM, 250 I). To remove the DNA from the C18, the beads were washed with 40% acetonitrile in water (101) and concentrated to ca 2 I on the Speedvac® or directly subjected to MALDI mass spectrometry.

Alternatively C18 beads were first covalently attached to a silicon surface (e.g. a silicon wafer) or adsorbed to a flat surface by hydrophobic interaction.

The results showed that acetonitrile/water at levels of ca. > 30% are enough to dissociate the hydrophobic interaction. Since the matrix used in MALDI contains 50% acetonitrile, the DNA can be released from the support and MALDIed successfully (with the trityl group removed during the MALDI process).

### EXAMPLE 4

### Attaching Beads to Silicon Chips

### Amino derivatization of silicon surface

Silicon wafers were washed with ethanol to remove surface debris and flamed over a bunsen burner until "red hot" to ensure oxidation of the surface. After cooling, the wafers were immersed in an anhydrous solution of 3-aminopropyltriethoxysilane in toluene (25%v/v) for 3 hours. The wafers were then washed with toluene (three times) then anhydrous dimethylacetamide (three times).

### Activation of Wang resin beads

Vacuum-dried Wang resin beads (5 g, 0.84 mmol/g loading, 4.2 mmol, diameter 100-200 mesh), obtained from Novabiochem, were suspended in pyridine (40 ml) with DMAP (0.1 eq, 0.42 mmol, 51 mg). To this was added succinic anhydride (5 eq, 21 mmol, 2.10 g) and the reaction was shaken for 12 hours at room temperature. After this time, the beads were washed with dimethylformamide (three times), then pyridine (three times) and suspended in pyridine/dimethylformamide (1:1, 20 ml). 4-Nitrophenol (2 eq, 8.4 mmol, 1.40 g) was added and the condensation was activated by adding dicyclohexylcarbodiimide (DCC) (2 eq, 8.4 mmol, 1.73 g) and the reaction mixture was shaken for 12 hours. The beads were then washed with dimethylformamide, pyridine and hexane, and stored at 4 C.

### Coupling of Beads to Silicon Wafers

The amino-derivatized silicon wafer is treated with a suspension of the 4-nitrophenol beads in dimethyl acetamide (DMA), and within five minutes, the beads are covalently linked to the surface. The coated surface can then be washed with DMA, ethanol and water, under which conditions the beads remain as a uniform monolayer. Care must be taken to avoid scratching the beaded surface. The beads can then be reacted with the amino-functionalized- modified DNA.

### EXAMPLE 5

### Immobilization of nucleic acids on solid supports via Streptavidin-Iminobiotin

2-iminobiotin N-hydroxy-succinimide ester (Sigma) was conjugated to the oligonucleotides with a 3'- or 5'-amino linker following the conditions suggested by the manufacture. The completion of the reaction was confirmed by MALDI-TOF MS analysis and the product was purified by reverse phase HPLC.

For each reaction, 0.1 mg of streptavidin-coated magnetic beads (Dynabeads M-280 Streptavidin from Dynal) were incubated with 80 pmol of the corresponding oligo in the presence of 1 M NaCI and 50 mM ammonium carbonate (pH 9.5) at room temperature for one hour. The beads with bound oligonucleotides were washed twice with 50 mM ammonium carbonate (pH 9.5). Then the beads were incubated in 2 *µ*l of 3-HPA matrix at room temperature for 2 min. An aliquot of 0.5 *µ*l of supernatant was applied to MALDI-TOF. For biotin displacement experiment, 1.6 nmol of free biotin (80 fold excess to the bound oligo) in 1 *µ*l of 50 mM ammonium citrate was added to the beads. After a 5 min. incubation at room temperature, 1 *µ*l of 3-HPA matrix was added and 0.5 *µ*l of supernatant was applied to MALDI-TOF MS. To maximize the recovery of the bound iminobiotin oligo, the beads from the above treatment were again incubated with 2 *µ*l of 3-HPA matrix and 0.5 *µ*l of the supernatant was applied to MALDI-TOF MS.

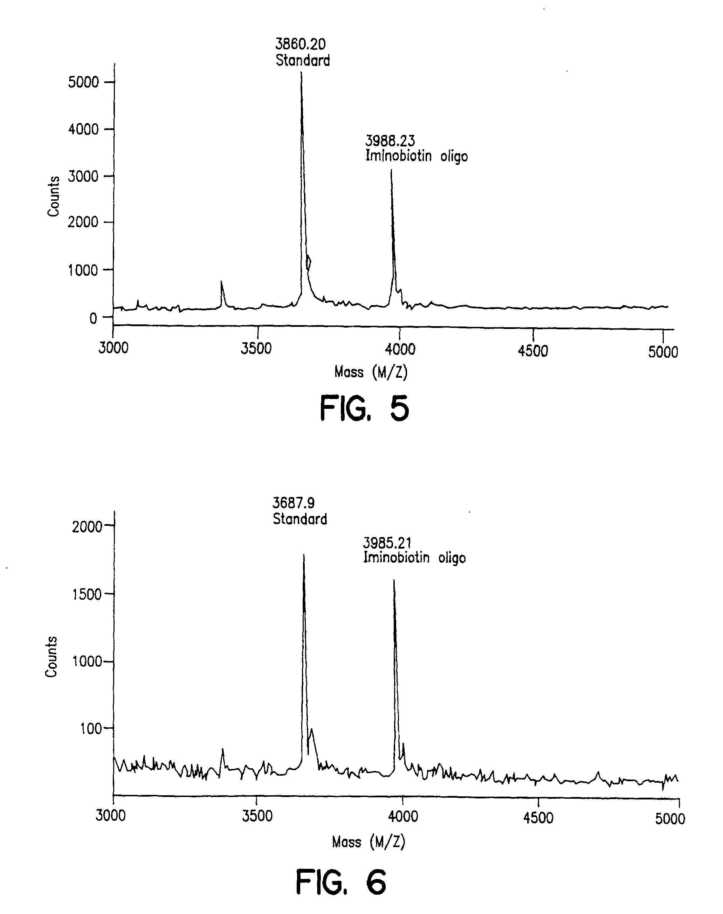

Matrix alone and free biotin treatment quantitatively released iminobiotin oligo off the streptavidin beads as shown in Figures 5 and 6. Almost no bound oligo was observed after the second treatment which confirmed the complete recovery.

### Equivalents

Those skilled in the art will recognize, or be able to ascertain using no more than routine experimentation, numerous equivalents to the specific procedures described herein. Such equivalents are considered to be within the scope of this invention and are covered by the following claims.

Since modifications will be apparent to those of skill in this art, it is intended that this invention be limited only by the scope of the appended claims.

## Claims

1. A composition, comprising a bead conjugated to a solid support and further conjugated to a macromolecule or a biological particle.

2. The composition of claim 1, wherein the macromolecule is selected from the group consisting of nucleic acids, peptides, proteins, amino acids and organic molecules.

3. A composition of claim 1, wherein the bead is conjugated to a macromolecule and the macromolecule is a nucleic acid.

4. A composition of any of claims 1-3, wherein the bead is made from a material selected from the group consisting of silica gel, glass, magnet, p-benzyloxybenzyl alcohol copolystyrene-divinyl benezene (DVB) resin, chlorotritylchloride copolystyrene-DVB resin, chloromethylated copolystyrene-DVB resin, metal, plastic, cellulose, cross-linked dextran, and agarose gel.

5. A composition of any of claims 1-3, wherein the bead is swellable.

6. A composition of any of claims 1-3, wherein the bead is nonswellable.

7. A composition of any of claims 1-3, wherein the bead is in the range of 1 to 100 µm in it largest dimension or diameter.

8. A composition of any of claims 1-3, wherein the solid support is in a form selected from the group consisting of capillaries, plates, membranes, wafers, combs, pins, wafers, wafers with arrays of pits and nanoliter wells.

9. A composition of any of claims 1-3, wherein the solid support is in the form of a bead, and conjugation of the bead to the solid support that is in the form of a bead is effected via ionic, covalent, hydrophobic, magnetic or polar interactions.

10. A composition of claim 3, wherein the nucleic acid is DNA.

11. A composition of claim 3, wherein the nucleic acid is RNA.

12. A process of making a bead conjugated to a solid support and further conjugated to a macromolecule or biological particle, comprising:
(a) conjugating a bead to a macromolecule or biological particle; and
(b) conjugating a bead to a solid support,
wherein steps (a) and (b) are performed sequentially in any order or simultaneously.

13. A process of claim 12, wherein the macromolecule is selected from the group consisting of nucleic acids, peptides, proteins, amino acids and organic molecules.

14. A process of claim 12, wherein the bead is conjugated to a macromolecule and the macromolecule is a nucleic acid.

15. A process of any of claims 12-14, wherein the bead is functionalized for linkage of macromolecules.

16. A process of any of claims 12-14, wherein the bead is functionalized with carboxy functional groups.

17. A process of any of claims 12 to 14, wherein the bead is functionalized with amino functional groups.

18. A process of any of claims 12-14, wherein the bead is conjugated to the macromolecule or biological particle prior to conjugation of the bead to the solid support.

19. A process of claim 12, wherein the bead is conjugated to the macromolecule or biological particle after the bead is conjugated to the solid support.

20. A kit comprising:
i) beads;
ii) an insoluble support; and
iii) conjugation means for linking molecules to the beads and the beads to the support.

## Patentansprüche

1. Zusammensetzung, umfassend ein Bead, welches an einen festen Träger konjugiert ist und weiter an ein Makromolekül oder ein biologisches Partikel konjugiert ist.

2. Zusammensetzung nach Anspruch 1, wobei das Makromolekül ausgewählt ist aus der Gruppe, bestehend aus Nucleinsäuren, Peptiden, Proteinen, Aminosäuren und organischen Molekülen.

3. Zusammensetzung nach Anspruch 1, wobei das Bead an ein Makromolekül konjugiert ist und das Makromolekül eine Nucleinsäure ist.

4. Zusammensetzung nach einem der Ansprüche 1-3, wobei das Bead aus einem Material ist, ausgewählt aus der Gruppe bestehend aus Silikagel, Glas, Magnet, p-Benzyloxybenzylalkoholcopolystyrol-divinylbenzol (DVB) -Harz, Chlortritylchloridcopolystyrol-DVB-Harz, chlormethyliertem Copolystyrol-DVB-Harz, Metall, Kunststoff, Cellulose, vernetztem Dextran und Agarosegel.

5. Zusammensetzung nach einem der Ansprüche 1-3, wobei das Bead quellbar ist.

6. Zusammensetzung nach einem der Ansprüche 1-3, wobei das Bead nicht quellbar ist.

7. Zusammensetzung nach einem der Ansprüche 1-3, wobei das Bead in seiner längsten Abmessung oder im Durchmesser im Bereich von 1 bis 100 µm liegt.

8. Zusammensetzung nach einem der Ansprüche 1-3, wobei der feste Träger eine Form aufweist, ausgewählt aus der Gruppe, bestehend aus Kapillaren, Platten, Membranen, Wafern, Kämmen, Stiften, Wafern, Wafern mit Vertiefungsgruppierungen und Nanoliter-Wells.

9. Zusammensetzung nach einem der Ansprüche 1-3, wobei der feste Träger in Form eines Beads vorliegt, und die Konjugation des Beads an den festen Träger in Form eines Beads mittels ionischer, kovalenter, hydrophober, magnetischer oder polarer Wechselwirkungen bewirkt ist.

10. Zusammensetzung nach Anspruch 3, wobei die Nucleinsäure DNA ist.

11. Zusammensetzung nach Anspruch 3, wobei die Nucleinsäure RNA ist.

12. Verfahren zur Herstellung eines Beads, welches an einen festen Träger konjugiert ist und weiter an ein Makromolekül oder ein biologisches Partikel konjugiert ist, umfassend:
(a) das Konjugieren eines Beads an ein Makromolekül oder ein biologisches Partikel, und
(b) das Konjugieren eines Beads an einen festen Träger,
wobei die Schritte (a) und (b) in beliebiger Reihenfolge nacheinander, oder gleichzeitig durchgeführt werden.

13. Verfahren nach Anspruch 12, wobei das Makromolekül ausgewählt wird aus der Gruppe, bestehend aus Nucleinsäuren, Peptiden, Proteinen, Aminosäuren und organischen Molekülen.

14. Verfahren nach Anspruch 12, wobei das Bead an ein Makromolekül konjugiert wird und das Makromolekül eine Nucleinsäure ist.

15. Verfahren nach einem der Ansprüche 12-14, wobei das Bead zur Bindung von Makromolekülen funktionalisiert ist.

16. Verfahren nach einem der Ansprüche 12-14, wobei das Bead mit Carboxyfunktionellen Gruppen funktionalisiert ist.

17. Verfahren nach einem der Ansprüche 12-14, wobei das Bead mit Aminofunktionellen Gruppen funktionalisiert ist.

18. Verfahren nach einem der Ansprüche 12-14, wobei das Bead vor Konjugation des Beads an den festen Träger an das Makromolekül oder das biologische Partikel konjugiert wird.

19. Verfahren nach Anspruch 12, wobei das Bead an das Makromolekül oder das biologische Partikel konjugiert wird, nachdem das Bead an den festen Träger konjugiert worden ist.

20. Kit, umfassend:
(i) Beads,
(ii) einen unlöslichen Träger, und
(iii) Konjugationsmittel zur Bindung von Molekülen an die Beads, und der Beads an den Träger.

## Revendications

1. Composition comprenant une bille conjuguée à un support solide et conjuguée en outre à une macromolécule ou une particule biologique.

2. Composition suivant la revendication 1, dans laquelle la macromolécule est choisie dans le groupe consistant en des acides nucléiques, des peptides, des protéines, des amino-acides et des molécules organiques.

3. Composition suivant la revendication 1, dans laquelle la bille est conjuguée à une macromolécule et la macromolécule consiste en un acide nucléique.

4. Composition suivant l'une quelconque des revendications 1 à 3, dans laquelle la bille est produite à partir d'une matière choisie dans le groupe consistant en le gel de silice, le verre, une matière magnétique, une résine d'alcool p-benzyloxybenzylique-copolystyrènedivinylbenzène (DVB), une résine de chlorure de chlorotrityle-copolystyrène-DVB, une résine de copolystyrène-DVB chlorométhylée, un métal, une matière plastique, la cellulose, le dextrane réticulé et un gel d'agarose.

5. Composition suivant l'une quelconque des revendications 1 à 3, dans laquelle la bille peut gonfler.

6. Composition suivant l'une quelconque des revendications 1 à 3, dans laquelle la bille est non apte au gonflement.

7. Composition suivant l'une quelconque des revendications 1 à 3, dans laquelle la bille a sa plus grande dimension ou son diamètre compris dans l'intervalle de 1 à 100 µm.

8. Composition suivant l'une quelconque des revendications 1 à 3, dans laquelle le support solide est sous une forme choisie dans le groupe consistant en des capillaires, des plaques, des membranes, des peignes, des broches, des tranches, des tranches avec des réseaux de creux et de puits de l'ordre d'un nanolitre.

9. Composition suivant l'une quelconque des revendications 1 à 3, dans laquelle le support solide est sous forme d'une bille, et la conjugaison de la bille au support solide qui est sous forme d'une bille est effectuée par des interactions ioniques, covalentes, hydrophobes, magnétiques ou polaires.

10. Composition suivant la revendication 3, dans laquelle l'acide nucléique consiste en ADN.

11. Composition suivant la revendication 3, dans laquelle l'acide nucléique consiste en ARN.

12. Procédé pour la préparation d'une bille conjuguée à un support solide et conjuguée en outre à une macromolécule ou particule biologique, comprenant les étapes consistant :
(a) à conjuguer une bille à une macromolécule ou particule biologique ; et
(b) à conjuguer une bille à un support solide,
dans lequel les étapes (a) et (b) sont mises en oeuvre successivement dans n'importe quel ordre ou bien simultanément.

13. Procédé suivant la revendication 12, dans lequel la macromolécule est choisie dans le groupe consistant en des acides nucléiques, des peptides, des protéines, des amino-acides et des molécules organiques.

14. Procédé suivant la revendication 12, dans lequel la bille est conjuguée à une macromolécule et la macromolécule est un acide nucléique.

15. Procédé suivant l'une quelconque des revendications 12 à 14, dans lequel la bille est fonctionnalisée pour la liaison de macromolécules.

16. Procédé suivant l'une quelconque des revendications 12 à 14, dans lequel la bille est fonctionnalisée avec des groupes à fonctionnalité carboxy.

17. Procédé suivant l'une quelconque des revendications 12 à 14, dans lequel la bille est fonctionnalisée avec des groupes à fonctionnalité amino.

18. Procédé suivant l'une quelconque des revendications 12 à 14, dans lequel la bille est conjuguée à la macromolécule ou particule biologique avant la conjugaison de la bille au support solide.

19. Procédé suivant la revendication 12, dans lequel la bille est conjuguée à la macromolécule ou particule biologique après la conjugaison de la bille au support solide.

20. Kit comprenant :
i) des billes ;
ii) un support insoluble ; et
iii) des moyens de conjugaison pour lier des macromolécules aux billes et pour lier les billes au support.


### SEQUENCE LISTING

(1) GENERAL INFORMATION
   (i) APPLICANT:
      (A) NAME: SEQUENOM, INC.
      (B) STREET: 11555 Sorrento Valley Road
      (C) CITY: San Diego
      (D) STATE: California
      (E) COUNTRY: USA
      (F) POSTAL CODE (ZIP) : 92121
   (i) INVENTOR/APPLICANT:
      (A) NAME: DAVID M. LOUGH
      (B) STREET:
      (C) CITY:
      (D) STATE:
      (D) COUNTRY: United Kingdom
      (E) POSTAL CODE (ZIP) :
   (i) INVENTOR/APPLICANT:
      (A) NAME: HUBERT KÖSTER
      (B) STREET: 1640 Monument Street
      (C) CITY: Concord
      (D) STATE: Massachusetts
      (D) COUNTRY: USA
      (E) POSTAL CODE (ZIP) : 01742
   (i) INVENTOR/APPLICANT:
      (A) NAME: DIRK REUTER
      (B) STREET:
      (C) CITY:
      (D) STATE:
      (D) COUNTRY: GERMANY
      (E) POSTAL CODE (ZIP) :
   (i) INVENTOR/APPLICANT:
      (A) NAME: SCOOT HIGGINS
      (B) STREET:
      (C) CITY:
      (D) STATE:
      (D) COUNTRY:
      (E) POSTAL CODE (ZIP) :
   (ii) TITLE OF INVENTION: COMPOSITIONS AND METHODS FOR IMMOBILIZING NUCLEIC ACIDS TO SOLID SUPPORTS
   (iii) NUMBER OF SEQUENCES: 2
   (iv) CORRESPONDENCE ADDRESS:
      (A) ADDRESSEE: Brown, Martin, Haller & McClain
      (B) STREET: 1660 Union Street
      (C) CITY: San Diego
      (D) STATE: CA
      (E) COUNTRY: USA
      (F) ZIP: 92101-2926
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: NONE
   (vi) CURRENT APPLICATION DATA:
      (A) APPLICATION NUMBER:
      (B) FILING DATE: 06-NOV-1997
      (C) CLASSIFICATION:
   (vii) PRIOR APPLICATION DATA:
      (A) APPLICATION NUMBER: 08/746,036
      (B) FILING DATE: 11/06/96
   (vii) PRIOR APPLICATION DATA:
      (A) APPLICATION NUMBER: 08/933,792
      (B) FILING DATE: 03/18/96
   (viii) ATTORNEY/AGENT INFORMATION:
      (A) NAME: Seidman, Stephanie L
      (B) REGISTRATION NUMBER: 33,779
      (C) REFERENCE/DOCKET NUMBER: 7352-2003PC
   (ix) TELECOMMUNICATION INFORMATION:
      (A) TELEPHONE: 619-238-0999
      (B) TELEFAX: 619-238-0062
      (C) TELEX:
(2) INFORMATION FOR SEQ ID NO:1:
   (i) SEQUENCE CHARACTERISTICS:
      (A) LENGTH: 12 base pairs
      (B) TYPE: nucleic acid
      (C) TOPOLOGY: unknown
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:1:
   TGCACCTGAC TC                                       12
(2) INFORMATION FOR SEQ ID NO:2:
   (i) SEQUENCE CHARACTERISTICS:
      (A) LENGTH: 12 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:2:
   CTGTGGTCGT GC                                       12